# EUROPÄISCHE PATENTSCHRIFT

(11) **EP 3 090 766 B1**
(45) Veröffentlichungstag und Bekanntmachung des Hinweises auf die Patenterteilung: **16.08.2017**
(21) Anmeldenummer: 15020070.7
(22) Anmeldetag: 06.05.2015
(51) Int. Cl.: A61M 1/12, A61M 1/10, A61B 17/00

(54) **VORRICHTUNG ZUR REGULIERUNG DER VITALEN PARAMETER DES HERZ-KREISLAUFSYSTEMS**
DEVICE FOR REGULATING THE VITAL PARAMETERS OF THE CARDIOVASCULAR SYSTEM
DISPOSITIF DE RÉGLAGE DES PARAMÈTRES VITAUX DU SYSTÈME CARDIOVASCULAIRE

(43) Veröffentlichungstag der Anmeldung: 09.11.2016
(73) Patentinhaber: Peter Osypka Stiftung, 79639 Grenzach-Wyhlen (DE)
(72) Erfinder: Osypka, Peter, 79639 Grenzach-Wyhlen (DE)

(56) Entgegenhaltungen:
- US-A1- 2002 173 742
- US-A1- 2006 178 550
- US-A1- 2008 109 069
- US-B1- 6 974 436

## Beschreibung

Die Erfindung betrifft eine im Herzen implantierbare Überwachungs-und Regulierungsvorrichtung bestehend aus einer Überwachungsvorrichtung (Sensor-Monitor-System) welche die Vitalparameter des Herz-Kreislaufsystems bestimmt und einer Regulierungsvorrichtung welche den Blutfluss bzw. den Blutdruck reguliert.

Erstmals kann mit der erfindungsgemäßen Vorrichtung erhöhter Blutdruck durch Beeinflussung des Blutflusses im Herzen therapiert werden.

Erkrankungen des Herz-Kreislauf-Systems, wie Herzinsuffizienz, koronare Herzerkrankung und Herzinfarkt, zählen zu den häufigsten Krankheiten und sind in Deutschland die Todesursache Nr. 1. Deshalb sind die rechtzeitige Vorbeugung, Erkennung und Behandlung besonders wichtig.

Ein häufiger Auslöser von Herz-Kreislauferkrankungen kann Bluthochdruck sein. Bluthochdruck (Hypertonie) zählt zu den Volkskrankheiten. Schätzungsweise 50 Prozent der Europäer sind davon betroffen. Im hohen Lebensalter sind in den Industrienationen die meisten Menschen von Bluthochdruck betroffen.

Der Blutdruck ist der Druck, der in den Gefäßen vorherrscht. Bei jedem Herzschlag wird Blut aus dem Herzen in die Blutgefäße gepumpt. Dabei übt das Blut von innen Druck auf die Gefäßwand aus. Abhängig von der Herzaktion, unterscheidet man zwei Blutdruck-Werte:
- Systolischer Blutdruck: Er entsteht in der Phase, in der sich das Herz zusammenzieht, der sogenannten Systole. Das Blut gelangt dabei aus dem Herzen zunächst in die Hauptschlagader (Aorta). Dabei entsteht eine Druckwelle, die sich dann weiter über die Gefäßwände der Arterien fortsetzt, sodass auch in weiter entfernten Körperregionen (z.B. den Armen und Beinen) ein Gefäßdruck messbar ist.
- Diastolischer Blutdruck: In der Diastole dehnt sich der Herzmuskel, um sich erneut mit Blut zu füllen. In den Gefäßen herrscht noch immer ein Druck, der jedoch niedriger ist als der systolische Blutdruck.

Die Behandlung des Blutdrucks erfolgt meist medikamentös mit oft großen Nebenwirkungen.

Die internationale Patentveröffentlichung WO2014052919 (ROX Medical) beschreibt eine Vorrichtung zur Blutdrucksenkung, die als Gefäßkoppler bezeichnet werden kann. Die Vorrichtung besteht aus distalen und proximalen Klammerelementen zum Einsetzen in die Vene bzw. die Arterie und aus einem Verbindungsstück zur Schaffung einer künstlichen Anastomose zwischen arterieller und venöser Blutbahn.
US20080109069 beschreibt die Möglichkeit Blut vom linken Ventrikel zum Koronar Sinus zu leiten um die Durchblutung des Herzmuskels zu verbessern. US20020173742 beschreibt die Moglichkeit Blutdruckunterschiede zwischen Ventrikeln auszugleichen.

Der vorliegenden Erfindung liegt die Aufgabe zugrunde ein System zu schaffen, welches auf von der Norm abweichende Vitalparameter des Herz-Kreislaufsystems wie. z. B. Blutdruck, Herzfrequenz, Herz Zeit Volumen sofort reagieren kann.

Die Aufgabe wird gelöst durch die Schaffung eines künstlichen Zugangs zwischen dem Myokard (Herzwand) des rechten Ventrikels über die Herzscheidewand (Septum cordiale) in den linkem Ventrikel, sodass Blut vom linken Ventrikel in den rechten Ventrikel umgeleitet werden kann. Dadurch verringert sich die Druckwelle vom linken Ventrikel in die Aorta und das Gefäßsystem.

Die vorliegende Erfindung betrifft somit gemäß Anspruch 1 eine im Herzen implantierbare Überwachungs-und Regulierungsvorrichtung bestehend aus einer Überwachungsvorrichtung welche die Vitalparameter des Herz-Kreislaufsystems überwacht; einer Regulierungsvorrichtung welche den Blutfluss bzw. den Blutdruck reguliert,
wobei die Regulierungsvorrichtung aus einem rohrförmigen flüssigkeitsdichten Schaft (5) besteht,
wobei der Schaft (5) einen inneren Hohlkanal (8) aufweist in den proximal ein Druckventil (10) eingefügt ist; wobei der Hohlkanal (8) den gesamten Schaft (5) in axialer Richtung durchzieht, , wobei der Schaft (5) zwei scheibenförmige Abschnitte (1) und (2), die distal angeordnet sind und zwei scheibenförmige Abschnitte (3) und (4), die proximal angeordnet sind, aufweist, sowie einen zentralen Abschnitt (50), der die distal angeordneten scheibenförmigen Abschnitte (1) und (2) mit den proximal angeordneten scheibenförmigen Abschnitten (3) und (4) verbindet, wobei die Länge des zentralen Abschnitts (50) dem Querdurchmesser des rechten Ventrikels entspricht und wobei der zentrale Abschnitt (50) eine Öffnung (6) mit einem Druckventil (9) aufweist;
wobei die Überwachungsvorrichtung aus mindestens einem Drucksensor (7) besteht der sich distal im Hohlkanal (8) der Regulierungsvorrichtung befindet;
dadurch gekennzeichnet dass ein Schubelement (11), bestehend aus einem rohrförmigen oder zylindrischen Kolben (12) und einer Schubstange (13), gleitbar und rotierbar im Hohlkanal (8) der Regulierungsvorrichtung derart montiert ist, dass durch Bewegung des Kolbens (12) in Längsrichtung des Hohlkanals (8) oder durch Rotation des Kolbens (12) die Öffnung (6) der Regulierungsvorrichtung teilweise oder vollständig verschließbar ist.

Der verwendete Ausdruck "scheibenförmig" beinhaltet auch verwandte Strukturen wie schirmförmig, Diskus-förmig oder Ellipsoid-förmig.

Die scheibenförmigen Abschnitte (1), (2), (3) und (4) haben einen größeren Durchmesser als der rohrförmige Schaft (5). Das Durchmesser-Verhältnis ist circa 1:3-1:4. Der Durchmesser der scheibenförmigen Abschnitte ist vorzugsweise ungefähr gleich.

Der distale Teil des rohrförmigen Schafts mit den Abschnitten (1) und (2) ist vorgesehen und geeignet zur Implantation im Ventrikel-Septum.

Der proximale Teil des rohrförmigen Schafts mit den Abschnitten (3) und (4) ist vorgesehen und geeignet zur Implantation im Myokard des rechten Ventrikels.

Die scheibenförmigen Abschnitte (1), (2), (3) und (4) dienen zur Fixierung der erfindungsgemäßen Vorrichtung im Ventrikel-Septum bzw. im Myokard des rechten Ventrikels. Der Durchmesser der scheibenförmigen Abschnitte ist so ausgelegt, dass die Vorrichtung nach ihrer Implantation in ihrer Position bleibt und ein Verrutschen verhindert wird.

Der zentrale Abschnitt (50) des rohrförmigen Schafts (5) wird in seiner Länge den anatomischen Gegebenheiten zwischen der Myokard des rechten Ventrikels und dem Ventrikel-Septum angepasst. Die maximale Länge entspricht dem Querdurchmesser des rechten Ventrikels.

Der zentrale Abschnitt (50) ist mit einer Öffnung (6) versehen, sodass dadurch ein Zugang zu dem rechten Ventrikel geschaffen wird. Durch diesen Zugang kann Blut in den rechten Ventrikel fließen.

Die Öffnung (6) ist mit einem Druckventil (9) versehen, beispielsweise mit einem Silikonventil. Solche Ventile sind im Handel erhältlich. Druckventil (9) ist ein Einwegventil. Es sperrt den Blutfluss vom rechten Ventrikel in den Hohlkanal (8) der Regulierungsvorrichtung.

Das Ventil (9) ist wichtig, es verhindert den Blutfluss aus dem rechten Ventrikel in den Brustraum, immer dann, wenn sich Kolben (12) des Schubelements (11) oberhalb der Öffnung befindet.

Der Blutfluss nach außen wird durch Ventil (10) verhindert, welches proximal in den Hohlkanal der Regulierungsvorrichtung eingefügt ist. Auch Ventil (10) ist ein Einwegventil. Es sperrt den Blutfluss vom Hohlkanal (8) der Regulierungsvorrichtung nach außen in den Brustraum.

Die Regulierungsvorrichtung besteht aus einem biokompatiblen und flexiblen Material. Jedes biokompatible Material ist denkbar aus dem sich ein Schaft mit einem Hohlkanal mit scheibenförmigen Erweiterungen formen lässt. Die Vorrichtung muss faltbar sein, sodass sie mit Hilfe eines Trokars eingeführt werden kann.

Geeignete Materialien sind beispielsweise Kunststoffe wie Silikon oder Polyurethan, Metalle, Memory Materialien wie beispielsweise Nitinoldrähte, Nitinollitzen, mit Kunststoff ummantelte Nitinolfäden oder Memory-Kunststofffäden, mit weiteren Metallen ummantelte Nitinoldrähte, z. B. mit Gold ummantelte Nitinoldrähte.

In einer Ausführungsform besteht die Einführungsvorrichtung vollständig oder teilweise aus biokompatiblem Kunststoff beispielsweise aus Silikon oder Polyurethan. Vorzugsweise besteht die gesamte Vorrichtung aus Silikon.

In einer bevorzugten Ausführungsform besteht die erfindungsgemäße Vorrichtung aus einem Geflecht aus Memory Material. Das Geflecht besteht beispielsweise aus Nitinoldrähten.

Der Schaft (5) muss mit einem flüssigkeitsdichten Material beschichtet sein, sodass ungewollt kein Blut vom linken in den rechten Ventrikel fließen kann. Der Blutfluss vom linken in den rechten Ventrikel geschieht gewollt und gesteuert.

Das Geflecht des rohrförmigen Schafts (5) ist vorzugsweise mit biokompatiblem Kunststoff, z.B. mit Silikon beschichtet. Eine solche Beschichtung verhindert, dass Blut in das Geflecht eindringt und sich dort verdickt und dass ungewollt Blut aus dem Schaft austreten kann. Außerdem verbessert die Beschichtung das Gleiten und Rotieren des Schubelements.

Besteht die Regulierungsvorrichtung aus einem mit Silikon beschichteten Nitinolgeflecht so ist die Öffnung (6) beispielsweise eine Aussparung in der Silikonbeschichtung in die das Druckventil eingefügt ist. Die Öffnung (6) kann auch ein kleiner Silikonzylinder sein, der in den zentralen Abschnitt (50) der Regulierungsvorrichtung eingefügt wird.

Das Schubelement (11) besteht aus einem rohrförmigen oder zylindrischen Kolben (12) beispielsweise aus Kunststoff. Das Schubelement kann auch aus einer gekapselten Metalllitze oder aus einer Metallwendel gefertigt werden. Der Kolben (12) ist mit einer Schubstange (13) versehen. Der Durchmesser des Kolbens entspricht dem Durchmesser des Hohlkanals (8) im Schaft (5) sodass der Kolben den Schaft (5) dicht verschließt, sodass kein Blut durch das Myokard nach außen dringen kann.

Der Kolben (12) des Schubelements (11) muss im Hohlkanal (8) des Schafts (5) gleiten und rotieren können und gleichzeitig den Schaft (5) im proximalen Bereich abdichten.

In einer Ausführungsform ist der Kolben (12) des Schubelements (11) distal abgeschrägt. Die Abschrägung ermöglicht ein Verschließen der seitlichen Öffnung zum rechten Ventrikel allein durch Rotation.

Die Schubstange (13) ist mit einer Steuer- und Antriebseinheit (16) verbunden.

Durch Verschieben des Kolbens (12) in Längsrichtung des Hohlkanals (8) vom proximalen Bereich des Hohlkanals zum distalen Bereich kann die Öffnung (6) teilweise oder vollständig verschlossen werden.

Zum Verschieben des Kolbens schiebt die Antriebseinheit die Schubstange entlang des Schafts (5) von proximaler zu distaler Position und betätigt dadurch den mit der Schubstange verbundenen Kolben.

Die Antriebseinheit kann auch eine Rotation der Schubstange und somit eine Rotation des Kolbens bewirken.
In einer Ausführungsform besteht das Schubelement aus Silikon und die Schubstange kann nach der gewünschten Einstellung abgekoppelt werden.

Die Steuerung des Schubelementes kann auch manuell vorgenommen werden. Die Schubstange kann abgekoppelt werden. Erforderliche spätere Nachjustierungen können über ein Portsystem erfolgen.

Die Antriebseinheit besteht aus einer elektronisch gesteuerten Lineareinheit oder aus einer mit einem Elektromotor angetriebenen Gewindestange.

Die Überwachungsvorrichtung besteht aus mindestens einem Drucksensor (7), der distal in den Hohlkanal (8) des Schafts (5) eingefügt ist. Der Drucksensor muss so platziert werden, dass die Bewegung des Schubelements nicht behindert wird. Der Drucksensor dient zur Erfassung des linksventrikulären Blutdrucks. Geeignete Drucksensoren sind im Handel erhältlich.
Ein vorteilhafter Drucksensor ist der von der Firma Osypka AG und dem Frauenhofer Institut entwickelte Sensor "HYPER IMS", der die Daten telemetrisch überträgt. (Technology Review, März 2009, Seite 16).

Weitere Sensoren können anwesend sein, beispielsweise ein Sensor zur telemedizinischen Überwachung von Blutparametern oder Sensoren zur Messung der Temperatur, Blutzucker, Thrombozyten etc. Durch die Sensoren werden Abweichungen vom jeweiligen Normalwert des Patienten ermittelt.

Die erfindungsgemäße Vorrichtung erlaubt aufgrund ihres Hohkanals (8) ein Durchschieben von Einführungskathetern, Elektroden, elektrischen Zuleitungen und implantierbaren medizinischen Geräten. Auch kann die Überwachungs-und Regulierungsvorrichtung über den Hohlkanal (8) mit weiteren Therapie-Vorrichtungen verbunden werden. Mögliche Therapie-Vorrichtungen werden in der Europäischen Patentanmeldung EP14002331.8 bzw. in der internationalen Anmeldung PCT/EP2014002915 beschrieben. EP14002331.8 bzw. PCT/EP2014002915 beschreiben eine Vorrichtung durch welche ein Zugang zum linken Ventrikel über den rechten Ventrikel erreicht werden kann.

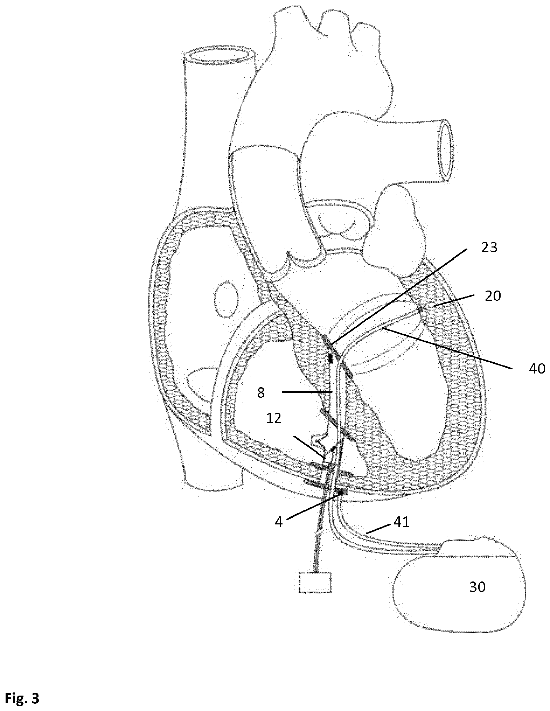

Falls eine links-ventrikuläre Stimulation gewünscht wird, wird mindestens eine Stimulationselektrode, beispielsweise eine Schraubelektrode (20) durch den Hohlkanal (8) und den Kolben (12) zum linken Ventrikel geführt. Geeignete Ventile, die ein Durchschieben von elektrischen Zuleitungen ermöglichen sind im Handel erhältlich. So wird eine Vorrichtung geschaffen, die nach Implantation im Herzen nicht nur den Blutdruck überwacht und reguliert, sondern gleichzeitig zur linksventrikulären Stimulation dient. (Fig. 3)

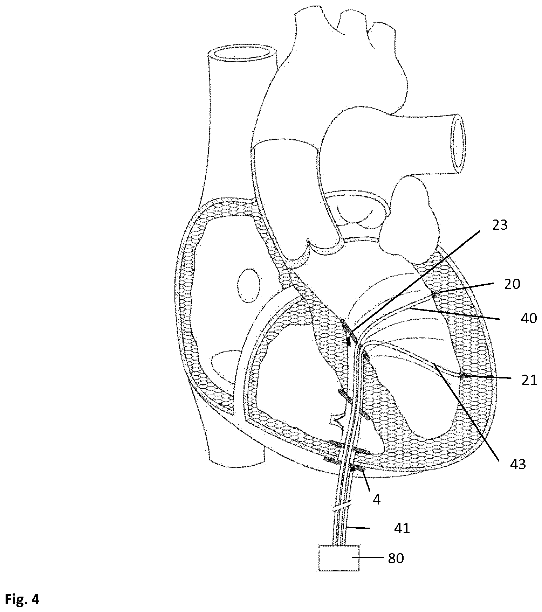

Durch Einführung von implantierbaren Elektroden in den linken Ventrikel (Fig. 4) kann man neben der Stimulation des Herzens auch die sich ändernde elektrische Impedanz von Systole und Diastole des Herzens messen und daraus die Herzleistung (Herzschlagvolumen, Strokevolumen, cardiac output) kalkulieren. US 6,511,438 Bernstein/Osypka beschreibt eine Methode zur Bestimmung des Herzschalgvolumens.

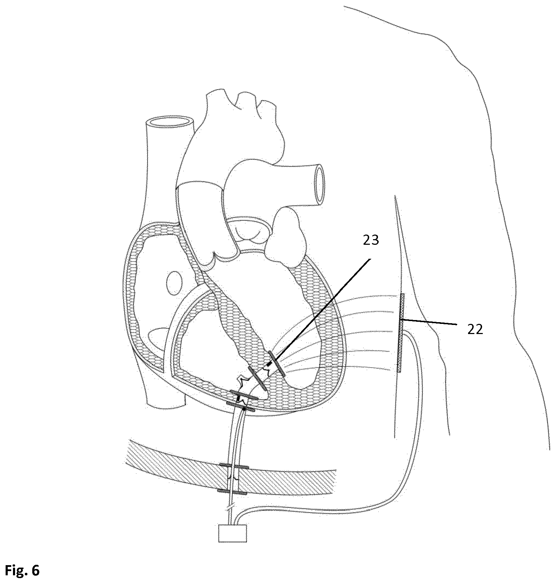

Zur Messung des der Herzimpedanz äquivalenten Widerstands kann auch eine Elektrode z.B. in Form einer Wendel außerhalb des Herzens im Brustraum oder außerhalb des Brustraums am Körpers positioniert werden, wie in (Fig. 6), (Fig. 7) dargestellt.
Als Mess-Strom zur Widerstandsmessung wird ein sinusförmiger Wechselstrom von über 10 kHz und einer Stromstärke von ca. 3 - 5 mA verwendet, der zwischen dem Pol (23) und den parallel geschalteten Polen (20) und (21) oder zwischen Pol (23) und Pol (22) fließt.

Der Herzschrittmacher kann unter der Bauchdecke implantiert sein oder außerhalb des Körpers sich befinden.

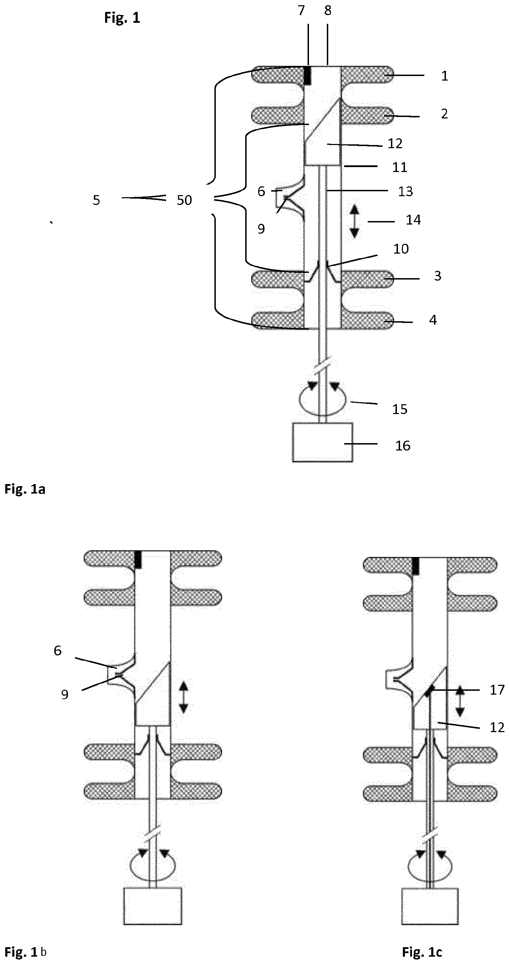

Besteht die Regulierungsvorrichtung aus einem Geflecht aus Nitinoldrähten, wird zur Herstellung der Vorrichtung zunächst ein rohrförmiger Schaft aus Drähten geflochten, wobei der Schaft an einem Ende eine kreisförmige oder ovale Öffnung trägt, am anderen Ende laufen die Drähte aus. Dieser geflochtene Schaft wird durch Wärmebehandlung und Zusammendrücken so verformt, dass sich daraus die in Fig. 1 dargestellten scheibenförmigen Abschnitte(1), (2), (3) und (4) bilden. Diese Abschnitte können als Einfach-Geflecht oder als Doppel-Geflecht geformt werden.

Die proximalen Drahtenden von Abschnitt (4) können in einer Kapsel gefasst werden, die ebenfalls eine Öffnung trägt, sodass der Hohlkanal (8) durchgehend ist. Das Nitinolgeflecht kann jedoch auch gegengleich in zwei Teilen geflochten werden und im zentralen Abschnitt verschweißt werden. Eine solche unmittelbare Verschweißung ist in WO2012095314 beschrieben.

Das Nitinolgeflecht liegt bei der Einführung mit Hilfe eines Trokars in einer zusammengefalteten Form vor. Wird das Geflecht aus dem Trokar geschoben entfaltet es sich.

Die erfindungsgemäße Vorrichtung ist gegebenenfalls auch als Dauerimplantat verwendbar. Es kann wie bereits bekannte transeptale Okkluder ins Gewebe einwachsen. Das Schubelement verschließt den Herzmuskel dicht, sodass eine Blutung verhindert wird.

**Die Bezifferung der Figuren ist:**
- 1,2,3,4: scheibenförmige Abschnitte
- 1,2: Doppelgeflecht distal
- 3,4: Doppelgeflecht proximal
- 5: rohrförmiger Schaft mit zentralem Abschnitt 50
- 6: Öffnung des rohrförmigen Schafts
- 7: Drucksensor
- 8: Hohlkanal
- 9: Ventil
- 10: Ventil
- 11: Schubelement
- 12: Kolben
- 13: Schubstange
- 14, 15: Pfeile
- 16: Antriebs-und Steuereinheit.
- 17: Sensor zur Überwachung von Blutparameter

- 20: Schraubelektrode
- 21: Schraubelektrode
- 22: Wendel
- 23: Pol
- 30: Herzschrittmacher
- 40, 41, 42,43: elektrische Zuleitung
- 50: zentraler Abschnitt von Schaft 5
- 51: Schaft
- 60: Einführungsvorrichtung
- 70: Bauchdecke
- 80: Impedanz-Messeinrichtung

### Beschreibung der Figuren

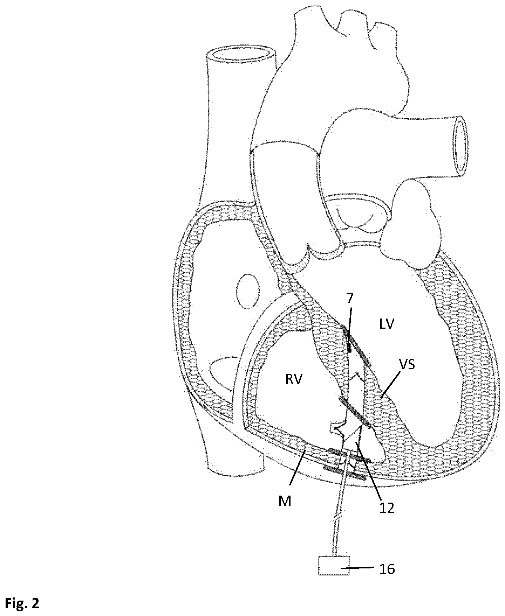

**In** **Fig. 1** zeigt die erfindungsgemäße Überwachungs-und Regulierungsvorrichtung, die aus einem Nitinolgeflecht besteht, mit zwei unterschiedlichen Positionen des Kolbens (12), (Fig. 1a und Fig. 1b)
   Die Vorrichtung besteht aus einem rohrförmigen Schaft (5), der aus Nitinoldraht geflochten ist und mit Silikon innen und außen beschichtet ist. Der zentrale Abschnitt (50) verbindet die distal angeordneten scheibenförmigen Abschnitte (1) und (2) mit dem proximal angeordneten scheibenförmigen Abschnitten (3) und (4). Der gesamte Schaft (5) wird in axialer Richtung von Hohlkanal (8) durchzogen. Der zentrale Abschnitt (50) besitzt eine Öffnung (6) in die ein Druckventil (9) eingefügt ist. Distal befindet sich der Drucksensor (7) zur Messung des linksventrikulären Blutdrucks. Der Drucksensor muss so platziert werden, dass die Bewegung des Schubelements (11) nicht behindert wird. Vorzugsweise befindet sich der Drucksensor (7) distal am oberen Ende von Hohlkanal (8). Proximal ist in Hohlkanal (8) ein Druckventil (10) eingefügt. Das Schubelement (11) besteht aus einem Kolben (12) der mit einer Schubstange (13) versehen ist. Die abkoppelbare Schubstange (13) ist mit der Steuereinheit (16) verbunden. Der Kolben (12) ist in den Hohlkanal (8) des Schafts (5) eingeschoben und verschließt den Schaft (5) dicht. Der Kolben (12) des Schubelements (11) ist in dieser Ausführungsform distal abgeschrägt. Pfeil (14) zeigt eine mögliche Bewegung des Kolbens (12) in Längsrichtung an. Pfeil (15) zeigt die mögliche Rotationsbewegung an.
**Fig.1a** zeigt die Ausgangsposition. Der Kolben (12) befindet sich distal oberhalb der Öffnung (6) und dichtet in Gebrauchsstellung nach Implantation der Vorrichtung den Zugang zum linken Ventrikel ab, sodass kein Blut fließen kann. Es kann kein Blut aus dem linken Ventrikel austreten. Der rechte Ventrikel ist durch Druckventil (9) verschlossen. Druckventil (10) verhindert das Blut nach außen in den Brustraum gelangen kann. Sensor (7) misst den Blutdruck. Werden Abweichungen von der Norm gemessen, wird der Kolben (12) durch die Steuereinheit (16) im Hohlkanal (8) nach unten geschoben bis die in **Fig.1b** dargestellte Position erreicht ist. Die Öffnung (6) zum rechten Ventrikel ist nun frei, sodass aufgrund des Druckunterschieds vom linken zum rechten Ventrikel Blut über Ventil (9) in den rechten Ventrikel fließen kann. Ventil (9) sperrt in eine Richtung vom rechten Ventrikel nach außen.
**Fig. 1c** zeigt eine Ausführungsform wobei ein weiterer Sensor (17) zur telemetrischen Analyse von Blutparametern (z. B. Hämoglobin, Thrombozyten, Blutzucker, etc.) sich auf dem Kolben (12) befindet Es ist auch denkbar, dass Sensor (17) distal im Hohlkanal (8) angebracht ist.
**Fig. 2** zeigt die in Fig. 1b dargestellte Überwachungs-und Regulierungsvorrichtung im Herz implantiert. Es gelten die Bezugszeichen der Fig. 1a, 1b. Nicht alle Bezugszeichen werden nochmals wiederholt.
   Der distale Teil des rohrförmigen Schafts mit den Abschnitten (1) und (2) ist im Ventrikel-Septum (VS) implantiert. Der proximale Teil des rohrförmigen Schafts mit den Abschnitten (3) und (4) ist im Myokard (M) des rechten Ventrikels implantiert. In der in Fig. 2 dargestellten Position des Kolbens (12) ist die Öffnung (6) freigelegt. Blut kann vom linken Ventrikel (LV) in den rechten Ventrikel (RV) gelangen aufgrund des Druckunterschieds in den Ventrikeln. Blut wird vom linken Ventrikel abgezogen und in den rechten Ventrikel umgeleitet. Dadurch verringert sich die Blutmenge im linken Ventrikel. Zieht sich das Herz nun zusammen gelangt weniger Blut in die Aorta. Der systolische Blutdruck sinkt.
   Wird der Kolben (12) des Schubelements (11) in distale Richtung zum Ventrikel Septum geschoben oder wird der Kolben (12) gedreht, so wird die Öffnung (6) verkleinert. Die Steuer-und Antriebseinheit (16) erlaubt jetzt abhängig vom durch Drucksensor (7) ermittelten linksventrikulären Blutdrucks eine Feinjustierung der Größe der Öffnung (6) und somit eine Justierung der umgeleiteten Blutmenge.
**Fig. 3** zeigt die implantierte Überwachungs-und Regulierungsvorrichtung, die gleichzeitig zur linksventrikulären Stimulation dient. Schraubelektrode (20) und die dazugehörige elektrische Zuleitung (40) werden über Hohlkanal (8) eingeführt und durch Ventil (10) (nicht eingezeichnet) und Kolben (12) hindurchgeführt. So wird die Schraubelektrode durch das Myokard des rechten Ventrikels und durch das Ventrikel-Septum geschoben. Die Schraubelektrode (20) wird beispielsweise im Endokard des linken Ventrikels eingeschraubt. Über die elektrische Zuleitung (40) wird die Schraubelektrode mit dem Herzschrittmacher (30) verbunden, der den Stimulationsimpuls abgibt. Auch der proximale Geflechtsabschnitt (4) der Überwachungs-und Regulierungsvorrichtung ist über Zuleitung (41) mit dem Herzschrittmacher verbunden, sodass das gesamte Nitinolgeflecht einen indifferenten Pol (23) bildet.
   Die Schraubelektrode kann sowohl als unipolare als auch als bipolare Elektrode ausgeführt werden. Bei der unipolaren Ausführung bildet entweder das Schrittmachergehäuse (30) den Gegenpol oder das Geflecht der Überwachungs- und Regulierungsvorrichtung (4) bildet den Gegenpol (23).
   Durch Einführung von implantierbaren Elektroden in den linken Ventrikel kann man neben der Stimulation des Herzens auch die sich ändernde elektrische Impedanz von Systole und Diastole des Herzens messen und daraus die Herzleistung (Herzschlagvolumen, Strokevolumen, cardiac output) kalkulieren.
**Fig. 4** zeigt die implantierte Überwachungs-und Regulierungsvorrichtung, die gleichzeitig zur Impedanz Messung dient. Die Schraubelektroden (20) und (21) und die dazugehörigen elektrischen Zuleitung werden über Hohlkanal (8) eingeführt und durch Ventil (10) (nicht eingezeichnet) und Kolben (12) (nicht eingezeichnet) hindurchgeführt. So werden die Schraubelektroden durch das Myokard des rechten Ventrikels und durch das Ventrikel-Septum geschoben. Die Schraubelektroden werden beispielsweise im Endokard des linken Ventrikels eingeschraubt. Über die elektrischen Zuleitungen (40) und (43) werden die Schraubelektroden mit dem Herzschrittmacher (30) bzw. mit dem Gerät zur Impedanz Messung (80) verbunden und dienen sowohl zur Stimulation als auch zur Impedanz Messung.
   Auch der proximale Geflechtsabschnitt (4) der Überwachungs-und Regulierungsvorrichtung ist über Zuleitung (41) mit dem Herzschrittmacher verbunden, sodass das gesamte Nitinolgeflecht einen indifferenten Pol (23) bildet. Die eingezeichneten Feldlinien zeigen den Verlauf des elektrischen Stromes während der Impedanz Messung. Für die elektrische Stimulation des linken Ventrikels genügt eine der beiden Schraubelektroden (20 oder 21). Auch hier kann die rechte Ventrikelspitze in der gleichen Weise stimuliert werden (Biventrikuläre Stimulation)(nicht eingezeichnet).
   Als Mess-Strom zur Widerstandsmessung wird ein sinusförmiger Wechselstrom von über 10 kHz und einer Stromstärke von ca. 3 - 5 mA verwendet, der zwischen dem Pol (23) und den parallel geschalteten Polen (20) und (21 fließt.

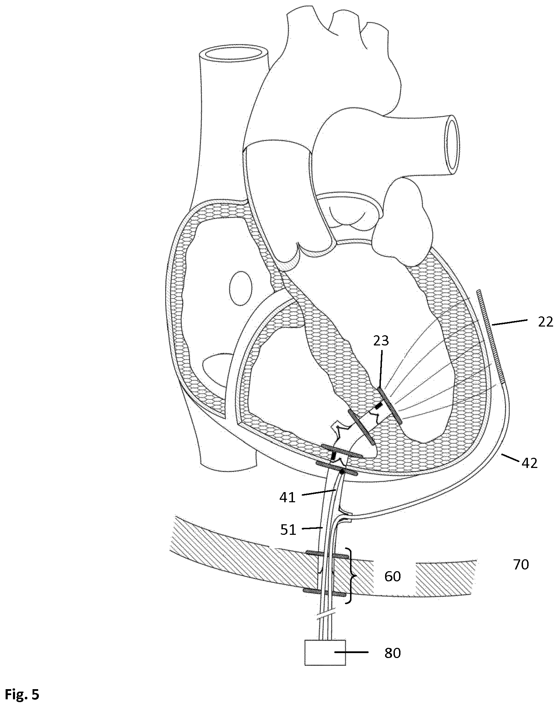

**Fig. 5** zeigt die implantierte Überwachungs-und Regulierungsvorrichtung ebenfalls zur Impedanz Messung geeignet. Ein zusätzlicher elektrischer Pol z.B. in Form einer Wendel (22) wird außerhalb des Herzens im Brustraum positioniert. Der Pol ist über Zuleitung (42) mit dem Herzschrittmacher bzw. mit dem Gerät zur Impedanz Messung (80) verbunden. Das Nitinolgeflecht ist über eine elektrische Zuleitung (41) mit dem Herzschrittmacher bzw. mit dem Gerät zur Impedanz Messung (80) verbunden und bildet den Pol (23). Als Mess-Strom wird ein sinusförmiger Wechselstrom von über 10 kHz und einer Stromstärke von ca. 3 - 5 mA verwendet, der zwischen den Polen (22) und (23) fließt.

Der Schrittmacher bzw Gerät (80) ist außerhalb des Körpers positioniert. Die elektrischen Zuleitungen (41), (42) zu den Polen (22), (23) werden durch die Einführungsvorrichtung (60), die über Schaft (51) an die erfindungsgemäße implantierte Überwachungs-und Regulierungsvorrichtung gekoppelt ist, durch die Bauchdecke (70) geführt. Die Einführungsvorrichtung (60) besteht auch aus einem Nitinolgeflecht und entspricht dem in Fig. 1a, 1b gezeigten Teil der Überwachungs-und Regulierungsvorrichtung mit den Geflechtsabschnitten (3) und (4). Schaft (51) hat eine Öffnung für Zuleitung (42).

Eine weitere Möglichkeit besteht darin, dass ein Pol (22) der Anordnung außerhalb des Brustraums am Körper wie in **(****Fig. 6****)** dargestellt, angebracht wird.

## Patentansprüche

1. Im Herzen implantierbare Überwachungs-und Regulierungsvorrichtung bestehend aus
einer Überwachungsvorrichtung welche die Vitalparameter des Herz-Kreislaufsystems misst und überwacht;
einer Regulierungsvorrichtung welche den Blutfluss bzw. den Blutdruck reguliert,
wobei die Regulierungsvorrichtung aus einem rohrförmigen flüssigkeitsdichten Schaft (5) besteht,
wobei der Schaft (5) einen inneren Hohlkanal (8) aufweist in den proximal ein Druckventil (10) eingefügt ist; wobei der Hohlkanal (8) den gesamten Schaft (5) in axialer Richtung durchzieht, wobei der Schaft (5) zwei scheibenförmige Abschnitte (1) und (2), die distal angeordnet sind und zwei scheibenförmige Abschnitte (3) und (4), die proximal angeordnet sind, aufweist, sowie einen zentralen Abschnitt (50), der die distal angeordneten scheibenförmigen Abschnitte (1) und (2) mit den proximal angeordneten scheibenförmigen Abschnitten (3) und (4) verbindet, wobei die Länge des zentralen Abschnitts (50) dem Querdurchmesser des rechten Ventrikels entspricht und wobei der zentrale Abschnitt (50) eine Öffnung (6) mit einem Druckventil (9) aufweist;
wobei die Überwachungsvorrichtung aus mindestens einem Drucksensor (7) besteht der sich distal im Hohlkanal (8) der Regulierungsvorrichtung befindet;
**dadurch gekennzeichnet dass** ein Schubelement (11) bestehend aus einem rohrförmigen oder zylindrischen Kolben (12) und einer Schubstange (13) gleitbar und rotierbar im Hohlkanal (8) der Regulierungsvorrichtung derart montiert ist, dass durch Bewegung des Kolbens (12) in Längsrichtung des Hohlkanals (8) oder durch Rotation des Kolbens (12) die Öffnung (6) der Regulierungsvorrichtung teilweise oder vollständig verschließbar ist.

2. Überwachungs-und Regulierungsvorrichtung gemäß Anspruch 1, wobei die Regulierungsvorrichtung aus einem Geflecht aus Nitinol besteht und Schaft (5) mit einem flüssigkeitsdichten Material beschichtet ist.

3. Überwachungs-und Regulierungsvorrichtung gemäß Anspruch 2, wobei das flüssigkeitsdichte Material Silikon ist

4. Überwachungs-und Regulierungsvorrichtung gemäß Anspruch 1 oder 2, wobei Kolben (12) mit einem Sensor bestückt ist.

5. Überwachungs-und Regulierungsvorrichtung gemäß Anspruch 1 oder 2, wobei Kolben (12) distal abgeschrägt ist.

6. Überwachungs-und Regulierungsvorrichtung gemäß Anspruch 1 oder 2, bestehend aus einer Überwachungsvorrichtung und einer Regulierungsvorrichtung wie in Anspruch 1oder 2 beschrieben und einer Stimulationsvorrichtung bestehend aus mindestens einer Stimulationselektrode (20) mit elektrischer Zuleitung (40) zu einem Schrittmacher (30), wobei die Stimulationselektrode (20) und die dazugehörige elektrische Zuleitung (40) über Hohlkanal (8) einführbar ist und durch Ventil (10) und Kolben (12) hindurchführbar ist.

7. Überwachungs-und Regulierungsvorrichtung gemäß Anspruch 1 oder 2, bestehend aus einer Überwachungsvorrichtung und einer Regulierungsvorrichtung wie in Anspruch 2 beschrieben und einer Vorrichtung zur Impedanz Messung bestehend aus mindestens einer Elektrode (22), die über eine elektrische Zuleitung mit einer Impedanz-Messeinrichtung (80) verbunden ist und wobei das Nitinolgeflecht über die elektrische Zuleitung (41) mit einer Impedanz-Messeinrichtung (80) verbunden ist und somit einen Pol darstellt, der gemeinsam mit der Elektrode (22) die Durchführung der temporären Impedanz Messung ermöglicht, um daraus die Herzleistung (Strokevolumen, Cardiac Output) des Patienten zu kalkulieren.

8. Überwachungs-und Regulierungsvorrichtung gemäß Anspruch 7, wobei Elektrode (22) außerhalb des Herzens im Brustraum oder außerhalb des Brustraums am Körper implantierbar ist.

9. Überwachungs-und Regulierungsvorrichtung gemäß Anspruch 7, wobei zwei Elektroden (20) und (21) im linken Ventrikel implantierbar sind und über die Zuleitung (40) und (41) mit der Impedanz-Messeinrichtung (80) verbunden sind.

## Claims

1. A monitoring and regulating device configured for implantation in the heart comprising
a monitoring device for measuring and monitoring the vital parameters of the cardiovascular system;
a regulating device for regulating the blood flow resp. the blood pressure, said regulating device consists of a tubular liquid-tight shaft (5) with an inner lumen (8) extending axially there through;
wherein a pressure valve (10) is inserted proximally into said lumen (8); said shaft (5) has two disc-shaped sections (1) and (2) which are arranged distally, two disc-shaped sections (3) and (4) which are arranged proximally and a central section (50) linking the distally arranged disc-shaped sections (1) and (2) with the proximally arranged disc-shaped sections (3) and (4); the length of central section (50) corresponds to the internal diameter of the right ventricle; said central section (50) has an opening (6) with a pressure valve (9);
said monitoring device consists of at least one pressure sensor (7) located distally in lumen (8) of the regulating device;
**characterized in that** a sliding element (11) consisting of a tubular or cylindrical piston (12) and a push rod (13) is inserted slidably and rotatably into lumen (8) of the regulating device in such a way that by moving piston (12) in the longitudinal direction of lumen (8) or by rotating piston (12), opening (6) of the regulating device can be closed partially or completely.

2. The monitoring and regulating device according to claim 1, wherein the regulating device consists of a nitinol braid and shaft (5) is coated with a liquid-tight material.

3. The monitoring and regulating device according to claim 2, wherein said liquid-tight material is silicon.

4. The monitoring and regulating device according to claim 1 or 2, wherein piston (12) has a sensor.

5. The monitoring and regulating device according to claim 1 or 2, wherein piston (12) has a tapered distal end.

6. The monitoring and regulating device according to claim 1 or 2, consisting of a monitoring and regulating device as described in claims 1 and 2 and of a stimulation device having at least one stimulation electrode (20) with electrical lead (40) to a pacemaker (30), wherein the stimulation electrode (20) and the associated electrical lead (40) can be inserted into lumen (8) and can be passed through valve (10) and piston (12).

7. The monitoring and regulating device according to claim 1 or 2, consisting of a monitoring and regulating device as described in claim 2 and of an impedance measuring device (80) with at least one electrode (22), said electrode being electrically connected to said impedance measuring device (80), wherein the nitinol braid is connected via electrical lead (41) to impedance measuring device (80) and thus said nitinol braid represents a pole which together with electrode (22) makes it possible to carry out the temporary impedance measurement in order to determine the cardiac output (Stroke volume) of the patient.

8. The monitoring and regulating device according to claim 7, wherein electrode (22) is implantable outside the heart in the chest cavity or outside the chest cavity on the body.

9. The monitoring and regulating device according to claim 7, wherein two electrodes (20) and (21) are implantable in the left ventricle and are connected to the impedance measuring device (80) via the lead (40) and (41).

## Revendications

1. Dispositif de surveillance et de régulation adapté pour être implanté dans le coeur, comprenant
un dispositif de surveillance pour mesurer et contrôler les signes vitaux du système cardiovasculaire et un dispositif de régulation pour réguler la circulation sanguine et la tension artérielle; dans lequel
le dispositif de régulation est constitué d' une tige tubulaire (5) étanche aux liquides, laquelle tige tubulaire a un lumen (8) dans lequel se trouve une soupape de pression (10) à l'extrémité proximale; lumen (8) traverse toute la tige dans la direction axiale,
lumen (8) a deux sections (1) et (2) en forme de disque qui sont positionnées à l'extrémité distale, deux sections (3) et (4) en forme de disque qui sont positionnées à l'extrémité proximale, et une section centrale (50) qui relie les section (1) et (2) avec les sections (3) and (4); la longueur de la section centrale (50) correspond au diamètre equatorial du ventricule droit; la section centrale (50) a une ouverture (6) avec une soupape de pression (9);
le dispositif de surveillance comprend au moins un capteur de pression (7) qui se trouve a l'extrémité distale dans lumen (8) du dispositif de réglulation;
**caractérisé en ce que** un élément de poussée (11)- comprenant un piston (12) tubulaire ou cylindrique et une tige de poussée (13) - est positionné dans lumen (8) de façon coulissante et rotative telle sorte que l'ouverture (6) du dispositif de régulation est partiellement ou complètement obturable par le movement du piston (12) dans la direction longitudinal du lumen (8) ou par rotation du piston (12).

2. Dispositif de surveillance et de régulation selon la revendication 1, dans lequel le dispositif de régulation est constitué d'un treillis en nitinol et dans lequel la tige tubulaire est revêtue d'un matériau étanche aux liquides.

3. Dispositif de surveillance et de régulation selon la revendication 2, dans lequel le matériau étanche aux liquides est fabriqué à base de silicone.

4. Dispositif de surveillance et de régulation selon la revendication 1 ou 2, dans lequel le piston (12) comprend un capteur.

5. Dispositif de surveillance et de régulation selon la revendication 1 ou 2, dans lequel le piston (12) est biseauté de manière distale.

6. Dispositif de surveillance et de régulation selon la revendication 1 ou 2, comprenant un dispositive de surveillance et un dispositif de régulation comme celui-ci est décrit dans la revendication 1 ou 2 et un dispositif de stimulation qui comprend au moins une électrode de stimulation (20) avec une ligne électrique (40) à un stimulateur cardiaque (30), dans lequel l'électrode de stimulation (20) et la ligne électrique associé (40) peut être positionnée via lumen (8) est peut être guidée à travers la soupape (10) et piston (12).

7. Dispositif de surveillance et de régulation selon la revendication 1 ou 2, comprenant un dispositif de surveillance et un dispositif de régulation comme celui-ci est décrit dans la revendication 2 et un dispositif de mesure d'impédance qui comprend au moins une électrode (22) avec une ligne électrique au mesure d'impédance (80), et dans lequel le treillis en Nitinol est relié via la ligne électrique (41) avec un dispositif de mesure d'impédance (80) et le treillis représente donc un pôle qui permet ensemble avec l'électrode (22) de mesurer l'impédance temporaire pour calculer le débit cardiaque du patient (Stroke volume).

8. Dispositif de surveillance et de régulation selon la revendions 7, dans lequel ladite électrode (22) peut être implantée à l'extérieur du coeur dans le thorax ou à l'extérieur de thorax au corps.

9. Dispositif de surveillance et de régulation selon la revendication 7, dans lequel deux électrodes (20) et (21) peuvent être implantées dans le ventricule gauche et peuvent être reliées via les lignes electriques (40) et (41) au dispositif de mesure d'impédance (80).
